# EUROPEAN PATENT SPECIFICATION

(11) **EP 1 497 451 B1**
(45) Date of publication and mention of the grant of the patent: **22.09.2010**
(21) Application number: 03721117.4
(22) Date of filing: 26.04.2003
(51) Int. Cl.: C12Q 1/02

(54) **METHOD AND DEVICE FOR DETECTING TOXIC MATERIAL IN WATER USING MICROBIAL FUEL CELL**
VERFAHREN UND VORRICHTUNG ZUM NACHWEIS VON TOXISCHEM MATERIAL IN WASSER UNTER VERWENDUNG EINER MIKROBIELLEN BRENNSTOFFZELLE
PROCEDE ET DISPOSITIF DE DETECTION DE MATIERES TOXIQUES DANS L'EAU A L'AIDE D'UNE CELLULE MICROBIENNE COMBUSTIBLE

(30) Priority: 27.04.2002 KR 2002023232
(43) Date of publication of application: 19.01.2005
(73) Proprietor: Korea Biosystems Corp., 136-791 Seoul (KR)
(72) Inventor: KIM, Hyung Joo, 130-010 Seoul (KR); CHOI, Dae Won, 120-111 Seoul (KR); HYUN, Moon Sik, 303-2403 Garak Ssangyong Apt., 138-162 Seoul (KR); NAM, Sung Hyun, 139-053 Seoul (KR)
(74) Representative: Beck, Michael Rudolf
(86) International application number: PCT/KR2003/000854
(87) International publication number: WO 2003/097861

(56) References cited:
- WO-A-01/04626
- EVANS MICHAEL R ET AL: "Biosensors for the measurement of toxicity of wastewaters to activated sludge" PESTICIDE SCIENCE, vol. 54, no. 4, December 1998 (1998-12), pages 447-452, XP002379862 ISSN: 0031-613X
- KONG Z ET AL: "An activated sludge-based biosensor for rapid IC-50 estimation and on-line toxicity monitoring" BIOSENSORS AND BIOELECTRONICS, vol. 8, no. 1, 1993, pages 49-58, XP002379863 ISSN: 0956-5663
- KIM B H ET AL: "A MICROBIAL FUEL CELL TYPE LACTATE BIOSENSOR USING A METAL-REDUCING BACTERIUM, SHEWANELLA PUTREFACIENS" JOURNAL OF MICROBIOLOGY AND BIOTECHNOLOGY, SEOUL, KR, vol. 9, no. 3, June 1999 (1999-06), pages 365-367, XP000862875 ISSN: 1017-7825
- HYUNG JOO KIM ET AL: "A MEDIATOR-LESS MICROBIAL FUEL CELL USING A METAL REDUCING BACTERIUM, SHEWANELLA PUTREFACIENS" ENZYME AND MICROBIAL TECHNOLOGY, STONEHAM, MA, US, vol. 30, no. 2, 14 February 2002 (2002-02-14), pages 145-152, XP001156325 ISSN: 0141-0229
- MASCINI M. ET AL.: 'DNA electrochemical biosensors' FRESENIUS' JOURNAL OF ANALYTICAL CHEMISTRY vol. 369, no. 1, 2001, pages 15 - 22, XP002999800
- CHITI G. ET AL.: 'Electrochemical DNA biosensor for environmental monitoring' ANALYTICA CHIMICA ACTA vol. 427, no. 2, 2001, pages 155 - 164, XP002985116
- MARRAZZA G. ET AL.: 'Disposable DNA electrochemical biosensors for environmental monitoring' ANALYTICA CHIMICA ACTA vol. 387, no. 3, 1999, pages 297 - 307, XP002985117
- CHAN C.M. ET AL.: 'Monitoring the toxicity of phenolic chemicals to activated sludge using a novel optical scanning respirometer' CHEMOSPHERE vol. 39, no. 9, 1999, pages 1421 - 1432, XP002985132
- LAYTON A.C. ET AL.: 'Validation of genetically engineered bioluminescent surfactant resistant bacteria as toxicity assessment tools' ECOTOXICOL. ENVIRON. SAF. vol. 43, no. 2, 1999, pages 222 - 228, XP002985118

## Description

### Technical Field

This invention relates to a detecting method of the toxic materials by a biological means and the device thereof. More specifically, this invention relates to an automatic detecting method of the toxic materials using microbial fuel cell and an automatic alarming device thereof.

### Background Art

Until now, the early detection and alarming device for the entry of the toxic materials in water has been developed by many researchers. There are chemical and biological detection devices in the conventional devices for determining toxic materials in water. The chemical detection device has limitation because of many materials present in water and only a few materials are quantitatively determined. It has the disadvantage of necessitating costly instruments and highly skilled engineers for the detection. In order to implement this limitation, various biological devices for detecting toxic materials present in water, have been developed.

Representative conventional biological detecting devices for toxic materials in water include monitoring methods by using fish, water flea and fluorescent microorganism. The device using fish for water quality monitoring takes advantage of the character of the fish to swim against the flow of water, namely it uses the countercurrent phenomenon for detection. After an anti-escape net is installed and when toxic materials are introduced through the inlet, the fish is effected and its swimming activity slows down. The effected fish is pushed back due to the current, yet the fish, by instinct, will move tail fin vigorously in order to move forward again, the tail fin touching the sensor during the process. This action is transformed into electric signals and recorded. The value of this electric signals are detected by the water quality monitoring device and is used to give alarm or in controlling the flow rate of water by the connected controller. This information is input/output through the monitor or the keyboard. The fish used for the purpose is usually a golden dace belonging to the dace genera in the carp family. The shortfalls of using the fish to detect the toxic material is that the object of detecting the toxicity is so large that it requires 8 hours to determine the toxicity when 8ppm phenol is introduced to water. The method of using fish for biological toxicity alarm system has low sensibility, and the detection time and the error range are wide. The selection and the growing condition of fish reduces reproducibility and uniformity of the alarming system. The device using water flea for the toxic material senses the activity of the water fleas by infrared sensor. It is based on the swimming activity of the fleas; 20 water fleas are placed into a glass or acrylic test chamber where water to be tested is introduced and discharged. The fleas react when water is introduced. While water without toxic materials is in, they show regular activity, yet if toxic materials are contained, their movement becomes irregular and vigorous. The more vigorous activity they show, the more frequently they hit the infrared sensor, making the electrical signal value increase. The temperature sensor determines the temperature all the time and the infrared sensor is controlled by the electronic controller, displaying the value through an output device. The detected water is discharged through water outlet. The early alarming device by water fleas is more sensitive than the device using the fish because the used object is smaller, but maintenance is difficult. When changing the fleas, the test chamber and the various tubes for the input/output of water are either washed or exchanged. Care and efforts are needed in growing the fleas, because the growing water should be prepared and exchanged for 2-3 times a week, and progeny and parent fleas should be carefully separated. The fleas are reared in a special growing chamber where its space is disinfected and any equipments interfering with the growth are eliminated. Fresh air must be supplied to the growing chamber.

The device for automatic detection of the toxicity in water, using the fixed fluorescent microorganism, determines the fluorescence against the toxicity. It needs various light detecting equipments, making it costly and requiring personnel for their maintenance and an expert for them as well.

The present invention is proposed based on the fact that the problems for such conventional automatic detection devices for water toxicity arise eventually from the sensor parts.

A method for detecting toxic materials in water by using a mediated amperometric biosensor is known from Evans, Michael R. et al.: "Biosensors for the measurement of toxicity of wastewaters to activated sludge" published in Pesticide Science, vol. 54, no. 4, December 1998, pages 447 to 452. More specifically, this reference discloses an activated sludge respiration inhibition test (ASRIT) and is related to a system monitoring toxicity by obtaining the signal of an electric current in response to the entry of toxic materials. It is characterised by using a 3-electrode system including a reference electrode, a counter electrode and a working electrode immobilizing an activated sludge or a lyophilised activated sludge to the surface of a working electrode with an anopore fixed-microorganism membrane. However, it uses p-benzoquinone as a redox mediator which can transfer an electron between the microorganisms and organic materials. Therefore, it may discharge secondary pollutants and it is difficult to keep signal stability due to incompleteness of the fixed-membrane. Further, it is difficult in maintenance of the activated sludge, and it must be replaced within a few months.

Kong, Z. et al.: "An activated sludge-based biosensor for rapid IC-50 estimation and online toxicity monitoring" published in Biosensors and Bioelectronics, vol. 8, no. 1, 1993, pages 49 to 58 disclose a Rapid Oxygen Demand and Toxicity tester (RODTOX) biosensor that utilizes a DO electrode and measures the respiration of micro-organisms by the amount of oxygen required. This reference also describes a MICROTOX sensor that measures the change in light of a luminescent bacterium.

### Disclosure of the Invention

The objective of the present invention is to solve the technical problems in the conventional automatic determination devices for detecting toxicity in water and to provide fast and correct determination method of toxicity with low cost and easy maintenance.

The objective of the present invention is accomplished by the system according to claim 1 and the method according to claim 4. Advantageous embodiments are laid down in further claims.

In the following, the present invention is illustrated in reference to the drawings. The present invention should be only understood within the scope of the claims and is not limited to the constitutions of the drawings in which:

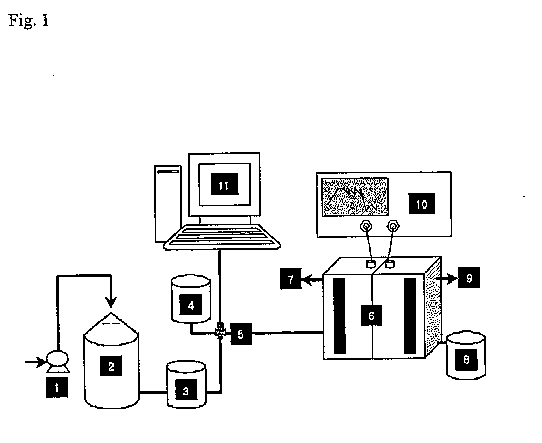

Figure 1 is a schematic illustration of the automatic determination device for toxic materials comprising a pump(1) taking a sample; a pretreatment tank(2) treating the sample; a microbial fuel cell(6) sensing the changes in the current occurred by the introduced toxic materials; and a PC and a controlling part(11) which control the value of when the signal is recognized.

The following illustration is the working mechanism of the device with the abovementioned constitution for determining toxic material by the use of the microbial fuel cell. The sample enters into the anode part after passing through the first and the second pretreatment tanks(2,3). The anode part is composed of a carbon felt and platinum(Pt) wire, while its inside part is filled usually with a microorganism catalyst which generates electrochemical energy using organic raw material. The cathode part is filled with ordinary water. Namely, the sample containing organic materials enters into the anode and the water-saturated with air enters the cathode. Now the organic materials are decomposed by microorganism at the anode part of the fuel cell and a current is generated. The current moves along the Pt wire and is measured by the voltmeter. In usual situation, the electric current does not show any range of changes, however, once the toxic material enters into the anode part, metabolism of the electrochemically active microorganism is slowed down, making the abrupt drop in the voltage. Such sharp drop of the current is processed by the PC and the controlling part, making the alarm activated in the audio/video display

### Brief Description of the Drawings

The above and other objects, features and other advantages of the present invention will be more clearly understood from the following detailed description taken in conjunction with the accompanying drawings, in which:
Figure 1 is a schematic diagram of the device for detecting the toxic materials.
Figure 2 is the graph showing the result of Embodiment 1.
Figure 3 is the graph showing the result of Embodiment 2.
Figure 4 is the graph showing the result of Embodiment 4.
Figure 5 is the graph showing the result of Embodiment 5.

****Description of parts list****

| | |
|---|---|
| (1) Sample inlet pump | (2) First pretreatment tank for sample |
| (3) Second pretreatment tank for sample | (4) Sample-gathering vessel |
| (5) Solenoid valve | (6) Microbial fuel cell |
| (7) Sample outlet | (8) Tap water chamber |
| (9) Tap water exit | (10) Voltmeter |
| (11) PC, controlling part | |

### Best Mode for Carrying Out Invention

### First preferred embodiment

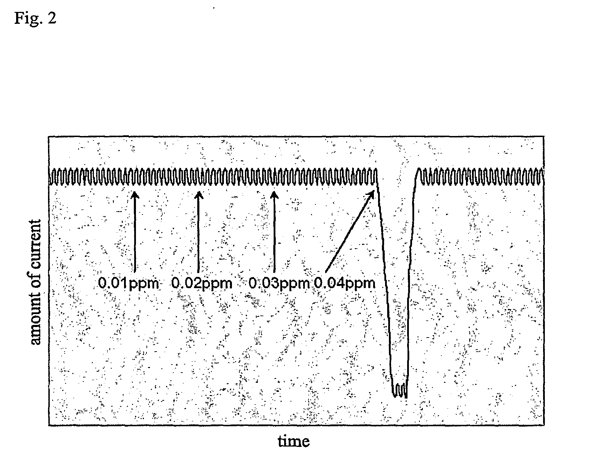

Active sludge was introduced into the anode part so that the electrochemically active bacteria in the sludge are attached to the electrode and densely cultured. To the cathode part, water saturated with air was incorporated, keeping a certain potential difference so as to make an efficient biological electrochemical reaction occur in the microbial fuel cell. Into this microbial fuel cell, added were glucose and glutamic acid(CODcr 200ppm, which means the chemical oxygen demand due to the potassium dichromate) as fuel and the generated current was measured by the volt meter(2000 multimeter, Keithley Instrument. Inc, USA) at 60 seconds interval, while adding to the used fuel cell, Cr⁶⁺ standard solution successively in the concentration of 0.01ppm, 0.02ppm, 0.03ppm, 0.04ppm and 0.05ppm. The result showed a sharp drop of electric current value which was usually generated in constant rate, at 0.04ppm of Cr^{6+.} (Fig. 2)

### Second preferred embodiment

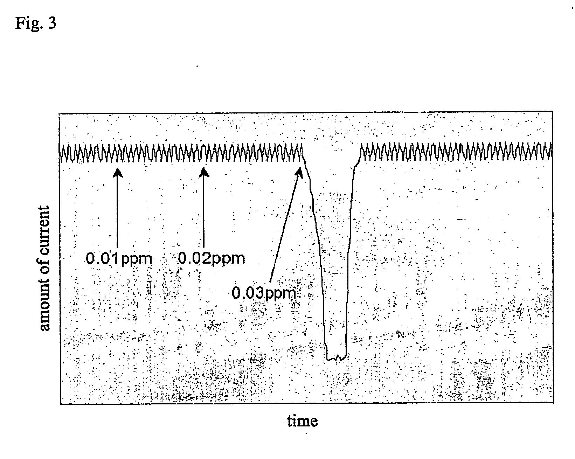

In the Embodiment 2, the same fuel cell and the fuel as in the Embodiment 1 was used. After feeding the fuel, the generated amount of current was measured by the volt meter(2000 multimeter, Keithley Instrument. Inc, USA) at 60 seconds interval, while adding to the used fuel cell, mercury(Hg) standard solution successively in the concentration of 0.01ppm, 0.02ppm, 0.03ppm, 0.04ppm and 0.05ppm. The result showed as in Embodiment 1 that the current generated in a constant rate showed a sharp drop at 0.03ppm ofHg. (Fig. 3)

### Third preferred embodiment

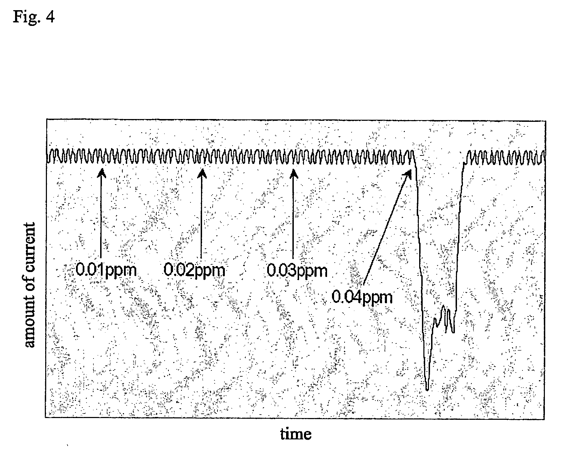

In the Embodiment 3, the same fuel cell and the fuel as in the Embodiment 1 was used. After feeding the fuel, the generated amount of current was measured by the volt meter(2000 multimeter, Keithley Instrument. Inc, USA) at 60 seconds interval, while adding to the used fuel cell, lead(Pb) standard solution successively in the concentration of 0.01ppm, 0.02ppm, 0.03ppm, 0.04ppm and 0.05ppm. The result showed as in Embodiment 1 that the current generated in a constant rate showed a sharp drop at 0.04ppm ofPb. (Fig. 4)

### Fourth preferred embodiment

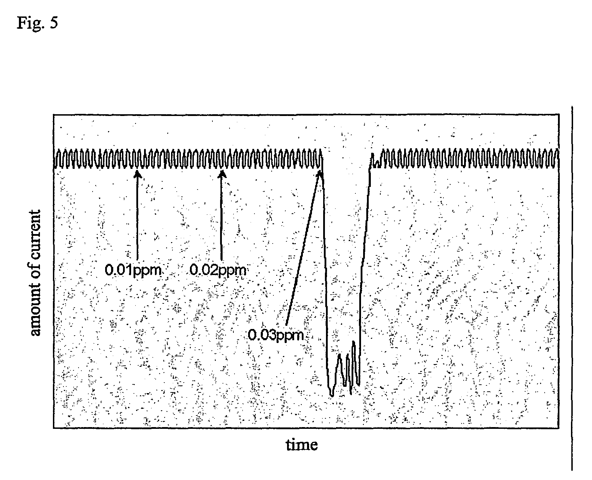

In the Embodiment 4, the same fuel cell and the fuel as in the Embodiment 1 was used. After feeding the fuel, the generated amount of current was measured by the volt meter(2000 multimeter, Keithley Instrument. Inc, USA) at 60 seconds interval, while adding to the used fuel cell, phenol standard solution successively in the concentration of 0.01ppm, 0.02ppm, 0.03ppm, 0.04ppm and 0.05ppm. The result showed as in Embodiment 1 that the current generated in a constant rate showed a sharp drop at 0.03ppm of phenol. (Fig. 5)

### Industrial Applicability

Thus according to the present invention, when the toxic materials are incorporated into the sample to be tested, there is an abrupt drop of the generated electricity by the electrochemically active bacteria in the microbial fuel cell, maximizing the sensitivity in detecting the toxic materials. Use of the microbial fuel cell minimizes the cost and the personnel in managing and maintaining the sensor part as well as remarkably enhancing the reproducibility and the degree of accuracy in detection of the toxic materials compared with the conventional alarming device. Once the entry signal of the toxic materials is sensed by the detecting device, the sample containing the toxic material is taken on the spot and kept in a sealed vessel. The sample is analyzed later quantitatively and qualitatively for tracing the cause of the entry and providing the data to forecast the consequent damage.

The present invention minimizes the damage by detecting the inflow of the toxic material in the early stage. Development of such excellent devices of detecting toxic materials contributes effectively to the national economy in that the related devices can be exported, replacing importation, once they are locally produced.

According to the present invention, the detecting device of the toxic materials using microbial fuel cell controls the degree of biological toxicity of the waste and sewage water and is used to detect rapidly the pollution of the intake water source of the drinking water. When the device is installed in the protected region of the water source, it can effectively prevent in advance the illegal disposal of the polluted materials by the industries and industrial complex facilities.

## Claims

1. System for automatically detecting toxic materials in water, comprising:
- a microbial fuel cell (6) having an anode part and a cathode part, the anode part being filled with active sludge containing an electrochemically active microorganism that is able to generate electrochemical energy by using organic material, and the cathode part being filled with tap water;
- a sample inlet pump (1) for taking a water sample and transferring it to the anode part; and
- a voltmeter (10) for measuring electrochemical voltage signals between the anode part and cathode part of the microbial fuel cell, said voltage signals representing electricity generated by said electrochemically active microorganism through decomposition of organic material contained in water samples provided by said pump;
- a computer and controlling part (11) which control the value of the signals and automatically determine the toxicity of the water sample in response to changes in the signals due to the entry of the toxic materials.

2. System according to claim 1, **characterised in that** a pretreatment tank (2) is arranged between the sample inlet pump (1) and the microbial fuel cell (6).

3. System according to claim 1 or 2, **characterised in that** a solenoid valve (5) is arranged between sample inlet pump (1) and the microbial fuel cell (6), and a sample gathering vessel (4) is connected to the solenoid valve (5), said solenoid valve (5) being adapted to change the flow of water sample to the sample gathering vessel (4) when the entry of toxic materials into the microbial fuel cell (6) is sensed.

4. Method for detecting toxic materials in water by using a microbial fuel cell (6) comprising an anode part and a cathode part, the method comprising the following steps:
- preparing a microbial fuel cell (6) having an anode part filled with activated sludge containing an electrochemically active microorganism that is able to generate electrochemical energy by using organic material, and a cathode part filled with tap water;
- taking a water sample;
- filtering out suspension and unwanted materials from the water sample;
- transferring the filtered water sample to the anode part of the fuel cell (6);
- measuring electrochemical voltage signals between the anode part and cathode part of the microbial fuel cell, said voltage signals representing electricity generated by said electrochemically active microorganism through decomposition of organic material contained in the water sample; and
- determining the degree of electrochemical voltage signal changes corresponding to the presence of toxic material from the microbial fuel cell (6) due to the entry of toxic materials.

5. Method according to claim 4, **characterised in that** the water sample is feed by a sample inlet pump (1) into the anode part of the microbial fuel cell (6), and that a PC and controlling part (11) controls the value of the voltage signals and automatically determines the toxicity.

6. Method according to claim 5, **characterised in that** a part of the flow of the water sample is feed into a sample-gathering vessel (4) which intakes and stores the water sample, by a solenoid valve (5) changing the flow of the water sample from the microbial fuel cell (6) to the sample-gathering vessel (4), when entry of toxic materials into the microbial fuel cell (6) is sensed.

## Patentansprüche

1. System zum automatischen Nachweis toxischen Materials in Wasser, umfassend:
- eine mikrobielle Brennstoffzelle (6) mit einem Anodenteil und einem Kathodenteil, wobei der Anodenteil mit aktivem Schlamm gefüllt ist, der einen elektrochemisch aktiven Mikroorganismus enthält, der in der Lage ist, unter Verwendung organischen Materials elektrochemische Energie zu erzeugen, und der Kathodenteil mit Leitungswasser gefüllt ist;
- eine Probenentnahmepumpe (1) zur Entnahme einer Wasserprobe und deren Einleitung in den Anodenteil, und
- ein Voltmeter (10) zur Messung elektrochemischer Spannungssignale zwischen dem Anodenteil und dem Kathodenteil der mikrobiellen Brennstoffzelle, wobei die genannten Spannungssignale elektrischen Strom darstellen, der vom genannten elektrochemisch aktiven Mikroorganismus durch Abbau organischen Materials erzeugt wurde, das in Wasserproben, die von der genannten Pumpe angefördert werden, enthalten ist;
- einen Rechen- und Steuerteil (11), das den Wert der Signale kontrolliert und automatisch aufgrund von Änderungen in den Signalen durch Einbringen toxischen Materials die Toxizität der Wasserprobe bestimmt.

2. System nach Patentanspruch 1, **dadurch gekennzeichnet, dass** ein Vorbehandlungsbehälter (2) zwischen der Probenentnahmepumpe (1) und der mikrobiellen Brennstoffzelle (6) angeordnet ist.

3. System nach Patentanspruch 1 oder 2, **dadurch gekennzeichnet, dass** ein Magnetventil (5) zwischen der Probenentnahmepumpe (1) und der mikrobiellen Brennstoffzelle (6) angeordnet ist und ein Probenaufnahmebehälter (4) mit dem Magnetventil (5) verbunden ist, wobei das genannte Magnetventil (5) derart angeordnet ist, dass es die Strömung von Wasserproben zum Probenaufnahmebehälter (4) ändert, wenn das Einbringen toxischen Materials in die mikrobielle Brennstoffzelle (6) festgestellt wird.

4. Verfahren zum Nachweis toxischen Materials in Wasser unter Verwendung einer mikrobiellen Brennstoffzelle (6), die ein Anodenteil und ein Kathodenteil aufweist, wobei das Verfahren die folgenden Schritte umfasst:
- Vorbereitung einer mikrobiellen Brennstoffzelle (6), die über einen Anodenteil verfügt, der mit aktiviertem Schlamm gefüllt ist, der einen elektrochemisch aktiven Mikroorganismus enthält, der in der Lage ist, unter Verwendung organischen Materials elektrochemische Energie zu erzeugen, und über einen Kathodenteil, der mit Leitungswasser gefüllt ist;
- Entnahme einer Wasserprobe;
- Ausfiltrieren suspendierten und unerwünschten Materials aus der Wasserprobe;
- Einleitung der filtrierten Wasserprobe in den Anodenteil der Brennstoffzelle (6);
- Messung elektrochemischer Spannungssignale zwischen dem Anodenteil und dem Kathodenteil der mikrobiellen Brennstoffzelle, wobei die genannten Spannungssignale elektrischen Strom darstellen, der vom genannten elektrochemisch aktiven Mikroorganismus durch Abbau organischen Materials erzeugt wurde, das in der Wasserprobe enthalten ist; und
- Bestimmung des Grades von Änderungen in den elektrochemischen Spannungssignalen aus der mikrobiellen Brennstoffzelle (6) aufgrund des Einbringens toxischen Materials, der der Anwesenheit toxischen Materials entspricht.

5. Verfahren nach Patentanspruch 4, **dadurch gekennzeichnet, dass** die Wasserprobe durch eine Probenentnahmepumpe (1) in den Anodenteil der mikrobiellen Brennstoffzelle (6) eingeleitet wird, und dass ein PC- und Steuerteil (11) den Wert der Spannungssignale kontrolliert und automatisch die Toxizität bestimmt.

6. Verfahren nach Patentanspruch 5, **dadurch gekennzeichnet, dass** ein Teil der Strömung der Wasserprobe durch ein Magnetventil (5) in einen Probenaufnahmebehälter (4), der die Wasserprobe aufnimmt und behält, eingeleitet wird, das die Strömung der Wasserprobe von der mikrobiellen Brennstoffzelle (6) zum Probenaufnahmebehälter (4) ändert, wenn Einbringen toxischen Materials in die mikrobielle Brennstoffzelle (6) festgestellt wird.

## Revendications

1. Système pour détecter automatiquement des matières toxiques dans de l'eau comprenant :
- une cellule électrochimique microbienne (6) ayant une partie anode et une partie cathode, la partie anode étant remplie de boue activée contenant un micro-organisme actif électrochimiquement qui est capable de générer de l'énergie électrochimique en utilisant des matières organiques et la partie cathode étant remplie d'eau du robinet ;
- une pompe de prise d'échantillons (1) pour prendre un échantillon et le transférer à la partie anode et
- un voltmètre (10) pour mesurer les signaux de tension électrochimique entre la partie anode et la partie cathode de la cellule électrochimique, les dits signaux de tension représentant l'électricité générée par ledit micro-organisme actif électro-chimiquement par la décomposition des matières organiques contenues dans les échantillons d'eau fournis par ladite pompe ;
- un ordinateur et une partie de contrôle (11) qui contrôlent la valeur des signaux et qui déterminent automatiquement la toxicité de l'échantillon d'eau en réponse aux variations des signaux dûes à l'entrée des matières toxiques.

2. Système selon la revendication 1, **caractérisé en ce qu'**un réservoir de pré-traitement (2) est placé entre la pompe de prise d'échantillons (1) et la cellule électrochimique microbienne (6).

3. Système selon la revendication 1 ou 2, **caractérisé en ce qu'**une électrovanne (5) est placée entre la pompe de prise d'échantillons (1) et la cellule électrochimique microbienne (6) et qu'un réservoir de collecte d'échantillons (4) est relié à l'électrovanne (5), ladite électrovanne (5) étant adaptée pour changer le flux de l'échantillon d'eau au réservoir de collecte d'échantillons (4) lorsque l'entrée de matières toxiques dans la cellule électrochimique microbienne (6) est détectée.

4. Procédé pour détecter des matières toxiques dans de l'eau en utilisant une cellule électrochimique microbienne (6) comprenant une partie anode et une partie cathode, le procédé comprenant les étapes suivantes :
- préparation d'une cellule électrochimique microbienne (6) ayant une partie anode remplie de boue activée contenant un micro-organisme actif électrochimiquement qui est capable de générer de l'énergie électrochimique en utilisant des matières organiques et une partie cathode remplie d'eau du robinet ;
- prise d'un échantillon d'eau ;
- filtration de la suspension et des matières indésirables de l'échantillon d'eau ;
- transfert de l'échantillon d'eau filtré à la partie anode de la cellule électrochimique (6) ;
- mesure des signaux de tension électrochimique entre la partie anode et la partie cathode de la cellule électrochimique microbienne, lesdits signaux de tension représentant l'électri-cité générée par ledit micro-organisme actif électrochimique-ment par la décomposition des matières organiques contenues dans l'échantillon d'eau et
- détermination du degré des variations de signal de tension électrochimique provenant de la cellule électrochimique microbienne (6) en raison de l'entrée de matières toxiques qui correspond à la présence de matières toxiques.

5. Procédé selon la revendication 4, **caractérisé en ce que** l'échantillon d'eau est introduit dans la partie anode de la cellule électrochimique microbienne (6) par une pompe de prise d'échantillons (1) et qu'un ordinateur et une partie de contrôle (11) contrôlent la valeur des signaux de ension et déterminent automatiquement la toxicité.

6. Procédé selon la revendication 5, **caractérisé en ce qu'**une partie du flux de l'échantillon d'eau est introduite dans un réservoir de collecte d'échantillons (4) qui prend et stocke l'échantillon d'eau par une électrovanne (5) qui change le flux de l'échantillon d'eau de la cellule électrochimique microbienne (6) vers le réservoir de collecte d'échantillons (4) lorsque l'entrée de matières toxiques dans la cellule électrochimique microbienne (6) est détectée.
